# EUROPEAN PATENT SPECIFICATION

(11) **EP 0 749 949 B1**
(45) Date of publication and mention of the grant of the patent: **14.04.1999**
(21) Application number: 96304487.0
(22) Date of filing: 17.06.1996
(51) Int. Cl.: C07C 51/12, C07C 67/36

(54) **Process for the carbonylation of alkyl alcohols**
Verfahren zur Carbonylierung von Alkylalkoholen
Procédé de carbonylation d'alcools alkyliques

(30) Priority: 19.06.1995 GB 9512427
(43) Date of publication of application: 27.12.1996
(73) Proprietor: BP Chemicals Limited, London EC2M 7BA (GB)
(72) Inventor: Poole, Andrew David, Salt End, Hull HU12 8DS (GB)
(74) Representative: Perkins, Nicholas David

(56) References cited:
- EP-A- 0 573 189
- GB-A- 2 146 637

## Description

The present invention relates to a process for the carbonylation of alkyl alcohols and/or reactive derivatives thereof in the presence of a rhodium catalyst.

Carbonylation processes in the presence of rhodium catalysts are known and are described, for example, in US 3769329 and EP-0055618-A.

J. Molecular Catalysis, 39 (1987) 115-136 relates to addition of iodide salts to rhodium catalyst solutions for methanol carbonylation at low water concentrations (<2M) to promote the rate of carbonylation and stabilise the rhodium catalyst. The rate of methanol carbonylation in the presence of MnI₂ was reported together with the rates for a large number of other iodide salts at a total reaction pressure of 400 psig. We believe that under such conditions, the rate of the carbonylation reaction is not limited by the partial pressure of carbon monoxide. Furthermore, the molar ratio of manganese : rhodium in the reported experiment using manganese is very high and is estimated to be (> 90) : 1.

Operation of carbonylation processes at low partial pressure of carbon monoxide is desirable because it leads to an increased utilisation of the carbon monoxide reactant, for example, by reducing loss of carbon monoxide in the gases vented from the reactor.

A problem with rhodium-catalysed carbonylation reactions is that at low partial pressure of carbon monoxide in the carbonylation reactor, that is at less than or equal to 7 bar, the rate of the carbonylation may be limited by the partial pressure of carbon monoxide.

The technical problem to be solved is to provide a process for the carbonylation of an alkyl alcohol and /or a reactive derivative thereof in the presence of a rhodium catalyst and an alkyl halide which overcomes this problem.

Thus, according to the present invention there is provided a process for the carbonylation of an alkyl alcohol and/or a reactive derivative thereof which process comprises the steps of (i) contacting, in a carbonylation reactor, said alcohol and/or a reactive derivative thereof with carbon monoxide in a liquid reaction composition comprising (a) a rhodium catalyst, (b) alkyl halide, and (c) at least a finite concentration of water and (ii) recovering carbonylation product from said liquid reaction composition characterised in that there is present in the carbonylation and/or product recovery steps a partial pressure of carbon monoxide less than or equal to 7 bar and a stabiliser for the rhodium catalyst comprising manganese, at a molar ratio of manganese : rhodium of (0.2 to 20) : 1.

In the process of the present invention the use of a manganese stabiliser for the rhodium catalyst has a beneficial effect on the rate of rhodium-catalysed carbonylation of an alkyl alcohol and/or a reactive derivative thereof at a partial pressure of carbon monoxide of less than or equal to 7 bar partial pressure, which would be carbonylation rate limiting in the absence of the manganese stabiliser.

Thus according to one aspect of the present invention there is provided a process for the carbonylation of an alkyl alcohol and/or a reactive derivative thereof which process comprises contacting, in a carbonylation reactor, an alcohol and/or a reactive derivative thereof with carbon monoxide in a liquid reaction composition at a partial pressure of carbon monoxide in said reactor of less than or equal to 7 bar, in which the liquid reaction composition comprises (a) a rhodium catalyst, (b) alkyl halide, (c) at least a finite concentration of water and (d) a stabiliser for the rhodium catalyst comprising manganese in a form active for promotion of the carbonylation reaction at a molar ratio of manganese : rhodium of (0.2 to 20) : 1.

In the carbonylation process of the present invention it has been unexpectedly found that the manganese stabiliser has a beneficial effect on the carbonylation process at a much lower concentration than is described in J. Molecular Catalysis, 39 (1987) 115-136. The beneficial effect on the carbonylation reaction of manganese in the process of the present invention has been found to occur at a low partial pressure of carbon monoxide of less than or equal to 7 bar partial pressure, which would be carbonylation rate limiting in the absence of the manganese stabiliser whereas iodide salts such as lithium iodide have been found not to increase the carbonylation rate under such conditions.

Also, in the process of the present invention, the presence of a manganese stabiliser in the liquid reaction composition stabilises the rhodium catalyst when carbonylation product is separated from the rhodium catalyst and manganese in the liquid reaction composition during the product recovery step at a partial pressure of carbon monoxide less than that in the carbonylation reactor.

Thus, according to another embodiment of the present invention there is provided a process for the carbonylation of an alkyl alcohol and/or a reactive derivative thereof which process comprises the steps of (i) contacting, in a carbonylation reactor, said alcohol and/or a reactive derivative thereof with carbon monoxide in a liquid reaction composition comprising (a) a rhodium catalyst, (b) alkyl halide, and (c) at least a finite concentration of water; (ii) recovering said carbonylation product from said reaction composition at a partial pressure of carbon monoxide of less than or equal to 7 bar in the presence of a stabiliser for the rhodium catalyst comprising manganese, at a molar ratio of manganese : rhodium of (0.2 to 20): 1; and (iii) recycling from step (ii), said rhodium catalyst and said manganese stabiliser to said carbonylation step (i).

Suitable alkyl alcohols comprise C₁ to C₁₀, preferably C1 to C₆, more preferably C₁ to C₄ alkyl alcohols and yet more preferably methanol. Preferably, the alkyl alcohol is a primary or secondary alkyl alcohol. The product of the carbonylation of an alcohol having n carbon atoms and/or a derivative thereof is a carboxylic acid having n+1 carbon atoms and/or an ester of a carboxylic acid having n+1 carbon atoms and the alcohol having n carbon atoms. Thus the product of carbonylation of methanol and/or a derivative thereof is acetic acid and/or methyl acetate.

Suitable reactive derivatives of the alkyl alcohol include the corresponding alkyl ester of the alcohol and the corresponding carboxylic acid product, dialkyl ethers and alkyl halides, preferably iodides or bromides. Suitable reactive derivatives of methanol include methyl acetate, dimethyl ether and methyl iodide. A mixture of alkyl alcohol and reactive derivatives thereof may be used as reactants in the process of the present invention. Preferably, methanol and/or methyl acetate and/or dimethyl ether are used as reactants. At least some of the alkyl alcohol and/or reactive derivative thereof will be converted to, and hence present as, alkyl esters in the liquid reaction composition by reaction with carboxylic acid product or solvent. The concentration in the liquid reaction composition, of alkyl ester is suitably in the range 0.1 to 70% by weight, preferably 0.5 to 50% by weight, more preferably 0.5 to 35% by weight.

Water may be formed in situ in the liquid reaction composition, for example, by the esterification reaction between alkyl alcohol reactant and carboxylic acid product. Water may be introduced to the carbonylation reactor together with or separately from other components of the liquid reaction composition. Water may be separated from other components of reaction composition withdrawn from the reactor and may be recycled in controlled amounts to maintain the required concentration of water in the liquid reaction composition. Suitably, the concentration of water in the liquid reaction composition is in the range 0.1 to 15% by weight, preferably 1 to 15% by weight. Preferably, the concentration of water is maintained below 14%, more preferably below 11% and yet more preferably below 8% by weight.

The rhodium component of the catalyst in the liquid reaction composition may comprise any rhodium containing compound which is soluble in the liquid reaction composition. The rhodium component of the catalyst may be added to the liquid reaction composition for the carbonylation reaction in any suitable form which dissolves in the liquid reaction composition or is convertible to a soluble form. Examples of suitable rhodium-containing compounds which may be added to the liquid reaction composition include [Rh(CO)₂Cl]₂, [Rh(CO)₂I]₂, [Rh(Cod)Cl]₂, rhodium (III) chloride, rhodium (III) chloridetrihydrate, rhodium (III) bromide, rhodium (III) iodide, rhodium (III) acetate, rhodium dicarbonylacetylacetonate, RhCl₃(PPh₃)₃ and RhCl(CO)(PPh₃)₂.

Preferably, the rhodium catalyst concentration in the liquid reaction composition is in the range 50 to 5000 ppm by weight of rhodium, preferably 100 to 1500 ppm.

The manganese stabiliser may comprise any manganese-containing compound which is soluble in the liquid reaction composition. The stabiliser may be added to the liquid reaction composition for the carbonylation reaction in any suitable form which dissolves in the liquid reaction composition or is convertible to soluble form.

Examples of suitable manganese-containing compounds which may be used include Mn₂(CO)₁₀, manganese (II) acetate, manganese (III) acetate, manganese (II) bromide, manganese (II) bromide tetrahydrate, manganese (II) chloride, manganese (II) chloride hydrate, manganese (II) iodide, manganese (II) oxide, manganese (III) oxide, manganese (IV) oxide, Mn(CO)₅Br, Mn(CO)₅I.

It is believed that the manganese stabiliser is active for promotion of the carbonylation reaction when it comprises manganese in a low oxidation state such as Mn(0) and/or Mn(I). Thus, if the manganese is added to the reaction composition in a higher oxidation state, such as for example as Mn(II), it may not exhibit a promoting effect for the carbonylation reaction unless or until it is converted to a lower oxidation state, for example by contacting with a suitable reducing agent such as hydrogen.

The molar ratio of manganese stabiliser : rhodium catalyst is (0.2 to 20) :1, preferably (0.5 to 10) : 1.

Suitable alkyl halides have alkyl moieties corresponding to the alkyl moiety of the alkyl alcohol reactant and are preferably C₁ to C₁₀, more preferably C₁ to C₆ and yet more preferably C₁ to C₄ alkyl halides. Preferably the alkyl halide is an iodide or bromide, more preferably an iodide. Preferably, the concentration of alkyl halide in the liquid reaction composition is in the range 1 to 30%, preferably 1 to 20%, more preferably 2 to 16% by weight.

An iodide salt may also be present in the liquid reaction composition as a catalyst stabiliser. Such an iodide salt can be any metal iodide, quaternary ammonium iodide or quaternary phosphonium iodide salt. Preferably, the metal iodide is an alkali iodide or alkaline earth metal iodide, more preferably an iodide of lithium, sodium, potassium or cesium. Suitable quaternary ammonium iodides include quaternised amine, pyridine, pyrrolidine or imidazole for example N,N' dimethyl imidazolium iodide. Suitable quaternary phosphonium iodides include methyl tributyl phosphonium iodide, tetrabutyl phosphonium iodide, methyl triphenyl phosphonium iodide and the like. Such iodide salt stabilisers are described, for example in EP-A-0573189.

The carbon monoxide reactant may be essentially pure or may contain inert impurities such as carbon dioxide, methane, nitrogen, noble gases, water and C₁ to C₄ paraffinic hydrocarbons. The presence of hydrogen in the carbon monoxide and generated in situ by the water gas shift reaction is preferably kept low, for example, less than 1 bar partial pressure, as its presence may result in the formation of hydrogenation products. The partial pressure of carbon monoxide in the reactor and/or product recovery steps is less than or equal to 7 bar, under which conditions it has been found that the use of a manganese stabiliser according to the process of the present invention in the reactor has a beneficial effect on the carbonylation reaction by stabilising the catalyst and maintaining the rate of reaction.

The pressure of the carbonylation reaction is suitably in the range 10 to 200 barg, preferably 10 to 100 barg, more preferably 15 to 50 barg. The temperature of the carbonylation reaction is suitably in the range 100 to 300 °C, preferably in the range 150 to 220 °C, more preferably in the range 170 to 200 °C

Carboxylic acid and/or ester thereof may be used as a solvent for the reaction.

The process of the present invention may be performed as a batch or a continuous process, preferably as a continuous process.

The carboxylic acid and/or ester carbonylation product may be recovered from the liquid reaction composition by withdrawing liquid reaction composition from the reactor and separating the carbonylation product by one or more flash and/or fractional distillation stages from the other components of the liquid reaction composition such as rhodium catalyst, manganese stabiliser, alkyl halide, water and unconsumed reactants which may be recycled to the reactor to maintain their concentrations in the liquid reaction composition. The carbonylation product may also be removed as a vapour from the reactor.

In a preferred embodiment, liquid reaction composition comprising carboxylic acid product, rhodium catalyst, manganese stabiliser, alkyl halide, water, ester of the alkyl alcohol and the carboxylic acid product and unconsumed reactants is withdrawn from the reactor and passed to a flash separation zone at a total pressure less than of the carbonylation reactor, wherein with or without the addition of heat, vapour and liquid fractions are formed from the liquid reaction composition; the vapour fraction comprising carboxylic acid product, alkyl halide, water, unconsumed reactants and ester and the liquid fraction comprising carboxylic acid product, rhodium catalyst, manganese stabiliser, and water together with some alkyl halide and ester. The liquid fraction is recycled to the carbonylation reactor and the carboxylic acid product is recovered from the vapour fraction by one or more distillation stages, with the alkyl halide, water, ester and unconsumed reactants being recycled to the carbonylation reactor. The partial pressure of carbon monoxide in the flash separation zone is less than that in the carbonylation reactor, for example, less than 0.25 bar.

The invention will now be illustrated by way of example only by reference to the following examples.

In the Further Experiments described below manganese was found in the liquid reaction composition at the end of batch carbonylation without the addition of manganese stabiliser at a concentration of about 6 ppm, due presumably, to corrosion. This corresponded to a molar ratio of manganese : rhodium of (significantly less than 0.05) :1. It is therefore to be assumed that in the reported experiments without addition of manganese stabiliser, similar levels of manganese might be expected, that is a molar ratio of manganese : rhodium of (significantly less than 0.05) : 1.

The following experiments were performed using a 150 ml Hastelloy B2 (Trade Mark) autoclave equipped with a Magnedrive (Trade Mark) stirrer, liquid injection facility and cooling coils. A gas supply to the autoclave was provided from a ballast vessel, feed gas being provided to maintain the autoclave at a constant pressure. The rate of gas uptake at a certain point in a reaction run was used to calculate the carbonylation rate (with an accuracy believed to be +/-1%), as number of moles of reactant consumed per litre of cold degassed reactor composition per hour (mol/l/hr), at a particular reaction composition (reaction composition based on a cold degassed volume).

The methyl acetate concentration was calculated during the course of the reaction from the starting composition, assuming that one mole of methyl acetate is consumed for every mole of carbon monoxide that is consumed. No allowance was made for organic components in the autoclave headspace.

At the end of each experiment liquid and gas samples from the autoclave were analysed by gas chromatography.

For each batch carbonylation experiment the autoclave was charged with the manganese stabiliser (where applicable), the liquid components of the liquid reaction composition excluding part of the methyl acetate and/or acetic acid charge in which the rhodium catalyst was dissolved.

The autoclave was flushed twice with nitrogen and was then heated with stirring (1000 rpm) to 185°C. On attaining a temperature of 185°C nitrogen was introduced into the autoclave to achieve a desired pressure, less than the final reaction pressure. The gas feed lines were then vented free of nitrogen and filled with carbon monoxide. After allowing the system to stabilise for about 30 minutes, the rhodium catalyst dissolved in methyl acetate and/or acetic acid was injected into the autoclave using an over pressure of carbon monoxide. The autoclave pressure was subsequently maintained at a constant pressure by feeding carbon monoxide gas on demand from the ballast vessel through the liquid injection facility, this pressure being between 27 and 28 barg. The initial partial pressure of carbon monoxide employed in the experiment was then calculated by subtracting the observed pressure when nitrogen was introduced to the autoclave from the final reactor pressure.

Gas uptake from the ballast vessel was measured every 30 seconds and from this was calculated the rate of carbonylation. After uptake of carbon monoxide from the ballast vessel has ceased or the reaction had proceeded for a period of 40 minutes, whichever was sooner, the autoclave was isolated from the gas supply. The autoclave was subsequently cooled to room temperature and the gases in the head space of the autoclave were cautiously vented from the autoclave, sampled and analysed. The liquid reaction composition was discharged from the autoclave, sampled and analysed for liquid products and by-products.

To obtain a reliable baseline a number of identical baseline runs may have to be performed to condition the autoclave such that consistent rates are achieved. This conditioning period is often different from autoclave to autoclave and may depend upon its previous history.

In the batch autoclave experiments and examples, the concentrations of components changed during the experiment as the reaction proceeded. Thus, the methyl acetate derivative of the methanol reactant decreased in concentration as did the water concentration. Methyl iodide promoter concentration decreased slightly as the volume of the liquid reaction composition increased due to formation of carboxylic acid product. The initial methyl acetate concentration (about 18% by weight) was higher than might be expected to be used in a typical continuous process (for example about 0.1 to 5% by weight) which concentration would occur in the batch experiment as methyl acetate conversion progressed to completion.

The autoclave charges, carbon monoxide partial pressures and reaction pressures for Experiments A-G and Examples 1-3 are shown in Table 1.

The results of the analyses of the non-condensable gases vented at the end of the experiments are given in Table 2. Analyses of the liquid reaction compositions showed that acetic acid is the major product (>99%) in all cases. All reactions were undertaken at 185°C.

### Experiment A

A baseline experiment was performed at a relatively high water concentration ranging from 17.1 to 11.7% by weight during the course of the reaction. The initial partial pressure of carbon monoxide in the carbonylation reactor was 4.8 bar.

The reaction rate was calculated to be 3.6 mol/l/hr after 5 minutes, based upon uptake of carbon monoxide. The reaction ceased after only 104 mmol of carbon monoxide had been fed from the ballast vessel. This corresponded to the carbonylation of 43% of the methyl acetate substrate. On opening the autoclave evidence of extensive catalyst precipitation was found.

This is not an example according to the present invention because no manganese stabiliser was added to the liquid reaction composition. This experiment showed that at low partial pressure of carbon monoxide in the carbonylation reactor, the rate of reaction was only 3.6 mol/l/hr after five minutes under these conditions and that the reaction ceased, presumably because of deactivation and/or instability of the rhodium catalyst in the carbonylation reactor. The rate of reaction was lower than that which might be expected ( about 7.5 to 8 mol/l/hr) under similar conditions, but without the addition of nitrogen to the autoclave, that is at a partial pressure of carbon monoxide of greater than 7 bar.

The presence of a catalyst precipitate at the end of the experiment indicated that, in a process in which carboxylic acid product is separated at reduced carbon monoxide partial pressure from the rhodium catalyst in the reaction composition, the stability of the rhodium catalyst could be low.

### Experiment B

A further baseline experiment was performed at a lower carbon monoxide partial pressures than that used in Experiment B.

The reaction rate was calculated to be 2.4 mol/l/hr after 5 minutes, based upon uptake of carbon monoxide. The reaction ceased after only 56 mmol of carbon monoxide had been fed from the ballast vessel. This corresponded to carbonylation of 23% of the methyl acetate substrate. On opening the autoclave there was evidence of significant catalyst precipitation.

This is not an example according to the present invention because no manganese stabiliser was added to the liquid reaction composition. This again indicated a low rate of reaction, early cessation of the reaction and the potential for catalyst instability during separation of carboxylic acid product from rhodium catalyst.

### Example 1

Experiment B was repeated except that Mn₂(CO)₁₀ (1.97 mmol) was also charged to the autoclave.

The carbonylation rate after 5 minutes was calculated to be 8.0 mol/l/hr. The rate of uptake of carbon monoxide (mol/hr) from the ballast vessel was constant until more than 90% of the methyl acetate had been consumed during the course of the reaction (based on carbon monoxide uptake). Furthermore, there was no evidence of catalyst precipitation on opening the autoclave at the end of the reaction.

This example is according to the present invention since it demonstrated that addition of manganese stabiliser Mn₂(CO)₁₀ to the carbonylation mixture at a low partial pressure of carbon monoxide increased the rate of carbonylation reaction and provided that the rate was maintained throughout the reaction implying increased stability of the rhodium catalyst in the reactor. Also, because conditions in the batch autoclave experiment were a more severe test of catalyst stability than those that might generally be expected during separation of carboxylic acid from the rhodium catalyst in the reaction composition (in particular the temperature and residence times are high), the lack of a precipitate indicated that the presence of the manganese stabiliser will stabilise the rhodium catalyst in a process where the carboxylic acid is separated from the rhodium catalyst in the reaction composition at a partial pressure of carbon monoxide less than that of the reactor.

### Example 2

Example 1 was repeated except that the reaction was performed at a constant pressure of 27.4 barg and an initial carbon monoxide partial pressure of 4.7 bar. The carbonylation rate after 5 minutes was calculated to be 7.5 mol/l/hr. The rate of uptake of carbon monoxide (mol/hr) from the ballast vessel was constant throughout the course of the reaction (run to completion based on uptake of carbon monoxide from the ballast vessel). Again, there was no evidence of catalyst precipitation on opening the autoclave at the end of the reaction.

This example is according to the present invention and further demonstrated that addition of manganese stabiliser Mn₂(CO)₁₀ to the carbonylation reaction composition afforded benefits of carbonylation rate promotion and catalyst stabilisation and the benefit of catalyst stabilisation during separation of the carboxylic acid product from the rhodium catalyst in the reaction composition.

### Experiment C

An experiment was performed at a lower water concentration than employed in Experiments A and B ranging from 5.1 to 0.5% by weight during the course of the reaction. The reaction was performed at a constant pressure of 27.3 barg and at a initial partial pressure of carbon monoxide of 4.3 bar.

The reaction rate was calculated to be 0.9 mol/l/hr after 5 minutes, based upon uptake of carbon monoxide. The reaction ceased after only 14 mmol of carbon monoxide had been fed from the ballast vessel. This corresponded to carbonylation of 6% of the methyl acetate substrate. On opening the autoclave evidence of extensive catalyst precipitation was found.

This is not an example according to the present invention because no manganese stabiliser was added to the liquid reaction composition. This experiment indicated that a low rate of reaction, early cessation of the reaction and the potential for catalyst instability occurred when the concentration of water in the liquid reaction composition and the partial pressure of carbon monoxide were low.

### Experiment D

Experiment C was repeated with the addition of lithium iodide (iodide salt stabiliser).

The reaction rate after 5 minutes was calculated to be 1.2 mol/l/hr which, within experimental error, is the same as in Experiment C. The reaction ceased after only 40 mmol of carbon monoxide had been consumed. This corresponded to carbonylation of 16% of the methyl acetate substrate. There was no evidence of catalyst precipitation an opening the autoclave at the end of the reaction.

This experiment is not an example according to the present invention since no manganese stabiliser was added to the liquid reaction composition. This experiment shows that, whilst lithium iodide stabilised the rhodium catalyst against precipitation, it did not provide the benefit of increasing the carbonylation rate under conditions of low partial pressure of carbon monoxide.

### Example 3

Experiment D was repeated except that manganese stabiliser Mn₂(CO)₁₀ (2.00 mmol) was added to the autoclave instead of the lithium iodide. The carbonylation rate after 5 minutes was calculated to be 4.9 mol/l/hr. The carbonylation rate decreased progressively during the course of the reaction and the reaction, which was still proceeding, was stopped after 40 minutes.

Some solid formation was observed at the end of the experiment, this was believed to be manganese salt rather than rhodium catalyst because it was different in character to typical rhodium precipitate observed in Experiments A and B.

This example is according to the present invention. It demonstrated that addition of manganese stabiliser Mn₂(CO)₁₀ to the carbonylation mixture at a low partial pressure of carbon monoxide increased the rate of carbonylation reaction.

Also, as previously discussed, lack of a rhodium catalyst precipitate indicated that the presence of the manganese stabiliser will stabilise the rhodium catalyst in a process where the carboxylic acid is separated from the rhodium catalyst at a partial pressure of carbon monoxide less than that in the reactor.

Furthermore, the example showed that manganese was superior to equimolar concentrations of LiI at low partial pressures of carbon monoxide.

### Experiment E

A baseline experiment was performed at a low water concentration (ranging from 5.1 to 0.5 wt%) as in Experiment C. However, no nitrogen was added to the autoclave prior to introduction of carbon monoxide so the partial pressure of carbon monoxide, though not calculated, was greater than 7 bar. The carbonylation rate after 5 minutes was 6.9 mol/l/hr. The carbonylation rate was not constant during the reaction, and decreased progressively until the reaction was stopped after 40 minutes. This indicated a progressive deactivation of the rhodium catalyst as the water concentration decreased during the experiment.

This is not an example according to the present invention because no manganese stabiliser was added to the liquid reaction composition.

### Experiment F

Experiment E was repeated except that lithium iodide (3.81 mmol) was added to the reaction composition. The carbonylation rate was not constant during the reaction, but decreased progressively from an initial rate of 6.8 mol/l/hr, measured after 5 minutes, until the reaction was stopped after 40 minutes.

This is not an example according to the present invention because no manganese stabiliser was added to the liquid reaction composition. This experiment demonstrated that addition of lithium iodide (a known carbonylation catalyst stabiliser) to the liquid reaction composition, did not overcome the disadvantage of decreasing rate of carbonylation (and hence catalyst activity) during the course of this experiment. Neither was the rate of carbonylation reaction after 5 minutes increased.

### Experiment G

Experiment E was repeated except that Mn₂(CO)₁₀ (1.95 mmol) was also charged to the autoclave.

The carbonylation rate during the reaction was not constant, but decreased progressively from an initial rate of 6.8 mol/l/hr, measured after 5 minutes, until the reaction was stopped after 40 minutes.

This is not an example according to the present invention because the partial pressure of carbon monoxide was greater than 7 bar. The experiment showed that under these conditions, manganese did not promote the carbonylation reaction.

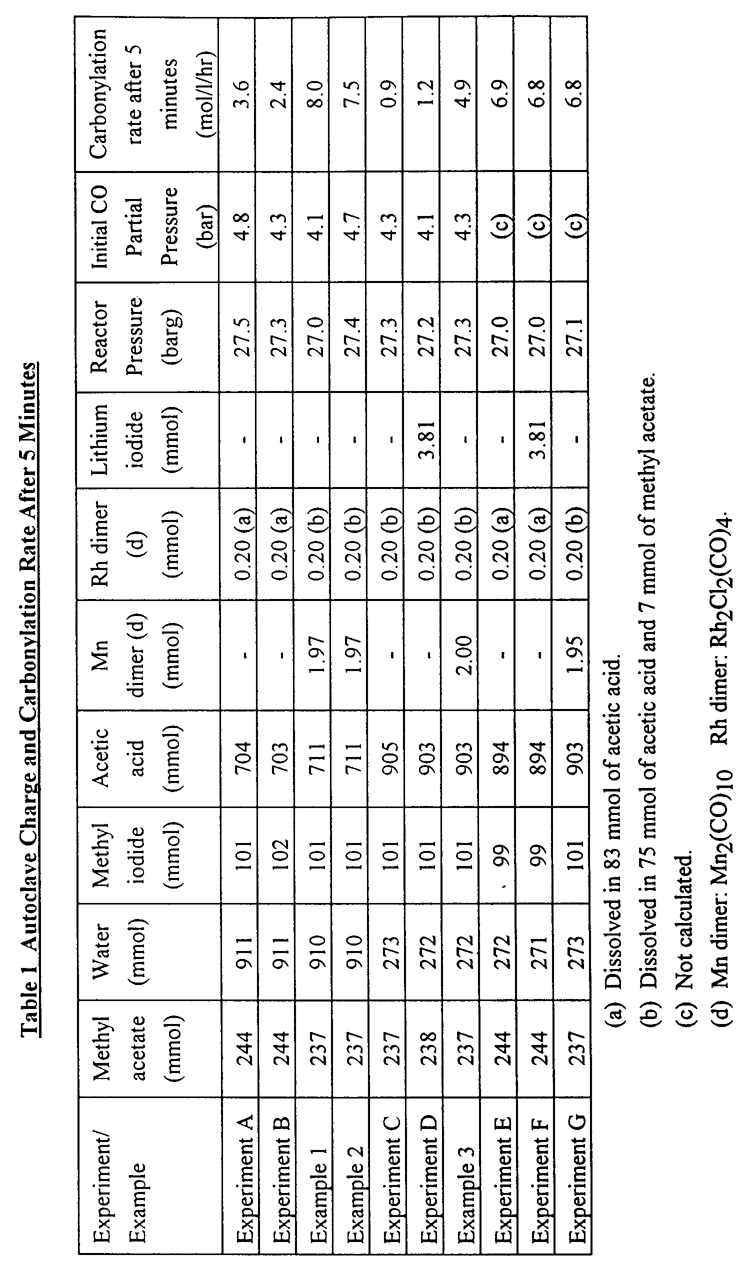

**Table 2**

| **Analyses of non-condensable gases** | | |
|---|---|---|
| | CH₄(% v/v) | CO₂(% v/v) |
| Experiment A | 9.8 | 4.4 |
| Experiment B | 9.5 | 3.8 |
| Example 1 | 5.1 | 2.3 |
| Example 2 | 8.5 | 7.7 |
| Experiment C | 0.4 | 0.5 |
| Experiment D | 2.4 | 0.7 |
| Example 3 | 4.7 | 1.0 |
| Experiment E | - | trace |
| Experiment F | - | trace |
| Experiment G | 4.0 | 0.8 |
| a. balance comprising hydrogen (not measured), nitrogen and carbon monoxide | | |

### Further Experiments

Experiments were performed in an analogous manner to those described in Experiments A-G and Examples 1-3, using a 300 ml Hastelloy B2 (Trade Mark). Gas uptake from the ballast vessel was measured every 2 seconds (rather than every 30 seconds as described previously) and from this was calculated the rate of carbonylation. Reactions were performed at constant pressures of between 26 and 28 barG.

The autoclave charges are given in Table 3 below.

**Table 3**

| **Autoclave Charges** | | | | | | | |
|---|---|---|---|---|---|---|---|
| Experiment/ Example | Methyl acetate (mmol) | Water (mmol) | Methyl iodide (mmol) | Acetic acid (mmol) | Mn (mmol) | Rh [1] (mmol) | Lithium iodide (mmol) |
| Experiment H | 474 | 545 | 203 | 1507 | - | 0.40 | 150 |
| Experiment I | 477 | 540 | 204 | 1506 | - | 0.40 | 149 |
| Example 4 | 475 | 547 | 203 | 1482 | 3.90 [2] | 0.40 | 149 |
| Example 5(a) | 475 | 547 | 203 | 1465 | 7.84 [3] | 0.40 | 150 |
| Example 5(b) | 476 | 546 | 206 | 1469 | 7.74 [3] | 0.40 | 149 |

| | | | | | | | |
|---|---|---|---|---|---|---|---|
| [1] Rh₂(CO)₄Cl₂ dissolved in 117 mmol acetic acid and 13 mmol methyl acetate. | | | | | | | |
| [2] Mn₂(CO)₁₀ | | | | | | | |
| [3] MnI₂ | | | | | | | |

### Experiment H

An experiment was performed in which lithium iodide was present in the liquid reaction composition at an initial concentration 10.4% by weight. The water concentration in the liquid reaction composition ranged from 5.1% to 0.5% by weight during the course of the reaction. The reaction was performed at a constant pressure of 28.2 barG and at an initial carbon monoxide partial pressure of 5.5 bar.

The reaction rate was calculated to be 2.6 mol/l/hr at a water concentration of 4.5% by weight. The reaction was stopped after only 11 mmol of carbon monoxide had been feed from the ballast vessel.

This is not an example according to the present invention because no manganese was added to the liquid reaction composition. This experiment showed that, even in the presence of a high concentration of lithium iodide, a poor reaction rate was obtained at a low carbon monoxide partial pressure.

### Experiment I

Experiment H was repeated except that hydrogen (1 bar) was introduced to the autoclave prior to warming to 185°C. The reaction was performed at an initial constant pressure of 26.1 barG and with an initial carbon monoxide partial pressure of 5.3 bar.

The reaction rate was calculated to be 3.5 mol/l/hr measured at a water concentration of 4.5% by weight. The reaction was stopped after 320 mmol of carbon monoxide had been consumed from the ballast vessel.

Experiment I is not according to the present invention but demonstrated that hydrogen had some limited beneficial effect on reaction rate at the low carbon monoxide partial pressure.

### Example 4

Experiment H was repeated with the addition of Mn₂(CO)₁₀ (3.90 mmol). The autoclave pressure was 27 barG and the initial partial pressure of carbon monoxide was 4.7 bar.

The reaction ran until completion. At a water concentration of 4.5% by weight, the carbonylation rate was be 8.0 mol/l/hr.

Example 4 is according to the present invention. It demonstrated that the addition of the manganese stabiliser Mn₂(CO)₁₀ enhanced the carbonylation rate at low carbon monoxide pressure in the presence of lithium iodide. Also, it showed that this manganese stabiliser was superior to the beneficial effect of hydrogen (1 bar measured at ambient conditions) in promoting this carbonylation reaction.

### Example 5

### Example 5(a)

Example 4 was repeated except that MnI₂ (7.84 mmol) was used as the manganese stabiliser. The reactor pressure was 28.5 barG and the partial pressure of carbon monoxide was initially 6.0 bar.

The reaction rate measured at a water concentration of 4.5% by weight was 2.0 mol/l/hr.

This experiment shows that this manganese (II) stabiliser was not effective in promoting the carbonylation reaction during the period of this batch autoclave reaction.

### Example 5(b)

Example 5(a) was repeated except that hydrogen (1 bar) was introduced to the autoclave prior to warming to reaction temperature. The reactor pressure was 26.5 barG and the initial partial pressure of carbon monoxide was calculated to be 5.1 bar.

The reaction rate was 4.2 mol/l/hr measured at a water concentration of 4.5% by weight.

This example is according to the present invention as it showed that hydrogen could be used to activate the manganese (II) stabiliser at the low carbon monoxide partial pressure. Furthermore, the reaction rate was superior to that which was observed in Experiment I.

## Claims

1. A process for the carbonylation of an alkyl alcohol and/or a reactive derivative thereof which process comprises the steps of (i) contacting, in a carbonylation reactor, said alcohol and/or a reactive derivative thereof with carbon monoxide in a liquid reaction composition comprising (a) a rhodium catalyst, (b) alkyl halide, and (c) at least a finite concentration of water and (ii) recovering carbonylation product from said liquid reaction composition characterised in that there is present in the carbonylation and/or product recovery steps a partial pressure of carbon monoxide less than or equal to 7 bar and a stabiliser for the rhodium catalyst comprising manganese, at a molar ratio of manganese : rhodium of (0.2 to 20) : 1.

2. A process as claimed in claim 1 in which the molar ratio of manganese : rhodium is (0.5 to 10): 1.

3. A process as claimed in claim 1 which comprises contacting, in a carbonylation reactor, an alcohol and/or a reactive derivative thereof with carbon monoxide in a liquid reaction composition at a partial pressure of carbon monoxide in said reactor of less than or equal to 7 bar, in which the liquid reaction composition comprises (a) a rhodium catalyst, (b) alkyl halide, (c) at least a finite concentration of water and (d) a stabiliser for the rhodium catalyst comprising manganese in a form active for promotion of the carbonylation reaction at a molar ratio of manganese : rhodium of (0.2 to 20) : 1.

4. A process as claimed in claim 3 in which the manganese stabiliser is present in the liquid reaction composition as manganese (I).

5. A process as claimed in claim 3 or claim 4 in which the liquid reaction composition comprises water at a concentration below 14 % by weight, preferably below 11 % by weight, more preferably below 8 % by weight.

6. A process as claimed in any one of claims 3 to 5 in which the liquid reaction composition further comprises an iodide salt stabiliser selected from the group consisting of metal iodides, quaternary ammonium iodides and quaternary phosphonium iodides.

7. A process as claimed in claim 6 in which the metal iodide salt stabiliser is selected from the group consisting of alkali iodides and alkaline earth metal iodides, preferably from the group consisting of iodides of lithium, sodium, potassium and cesium; the quaternary ammonium iodide is selected from the group consisting of a quaternised amines, pyridines, pyrrolidines and imidazoles, and is preferably N,N' dimethyl imidazolium iodide; and the quaternary phosphonium iodide is selected front the group consisting of methyl tributyl phosphonium iodide, tetrabutyl phosphonium iodide and methyl triphenyl phosphonium iodide.

8. A process as claimed in any one of claims 3 to 7 in which the molar ratio of manganese : rhodium in the liquid reaction composition is (0.5 to 10) : 1.

9. A process as claimed in any one of claims 3 to 8 in which the manganese stabiliser is converted to an active form by contacting with hydrogen.

10. A process as claimed in any one of the preceding claims which comprises contacting, in a carbonylation reactor, methanol and/or methyl acetate with carbon monoxide in a liquid reaction composition at a partial pressure of carbon monoxide in said reactor of less than or equal to 7 bar and in which the liquid reaction composition comprises (a) a rhodium catalyst at a concentration in the liquid reaction composition of 100 to 1500 ppm by weight of rhodium, (b) 2 to 16 % by weight methyl iodide, (c) less than 8 % by weight water, and (d) a stabiliser for the rhodium catalyst comprising manganese in a form active for promotion of the carbonylation reaction at a molar ratio of manganese stabiliser : rhodium catalyst of (0.5 to 10) : 1.

11. A process as claimed in claim 1 which comprises the steps of (i) contacting, in a carbonylation reactor, said alcohol and/or a reactive derivative thereof with carbon monoxide in a liquid reaction composition comprising (a) a rhodium catalyst, (b) alkyl halide, and (c) at least a finite concentration of water; (ii) recovering said carbonylation product from said reaction composition at a partial pressure of carbon monoxide of less than or equal to 7 bar in the presence of a stabiliser for the rhodium catalyst comprising manganese, at a molar ratio of manganese : rhodium of (0.2 to 20): 1; and (iii) recycling from step (ii), said rhodium catalyst and said manganese stabiliser to said carbonylation step (i).

12. A process as claimed in claim 11 in which the molar ratio of manganese : rhodium in step (ii) is (0.5 to 10) : 1.

13. A process as claimed in claim 11 or claim 12 in which the partial pressure of carbon monoxide in step (ii) is less than 0.25 bar.

14. A process as claimed in any one ofthe preceding claims in which the alkyl alcohol comprises methanol and the carbonylation product comprises acetic acid.

## Patentansprüche

1. Verfahren zur Carbonylierung eines Alkylalkohols und/oder eines reaktiven Derivats davon, wobei das Verfahren die Schritte umfaßt: (i) Inkontaktbringen des Alkohols und/oder eines reaktiven Derivats davon mit Kohlenmonoxid in einer flüssigen Reaktionszusammensetzung, umfassend (a) einen Rhodiumkatalysator, (b) Alkylhalogenid, und (c) mindestens eine endliche Konzentration an Wasser in einem Carbonylierungsreaktor, und (ii) Gewinnen des Carbonylierungsprodukts aus der flüssigen Reaktionszusammensetzung, dadurch gekennzeichnet, daß in den Carbonylierungs- und/oder Produktgewinnungsschritten ein Kohlenmonoxid-Partialdruck von weniger als oder gleich 7 bar und ein Mangan umfassender Stabilisator für den Rhodiumkatalysator bei einem Molverhältnis von Mangan: Rhodium von (0,2 bis 20):1 vorliegt.

2. Verfahren nach Anspruch 1, wobei das Molverhältnis Mangan:Rhodium (0,5 bis 10):1 ist.

3. Verfahren nach Anspruch 1, umfassend in einem Carbonylierungsreaktor Inkontaktbringen eines Alkohols und/oder eines reaktiven Derivats davon mit Kohlenmonoxid in einer flüssigen Reaktionszusammensetzung bei einem Kohlenmonoxid-Partialdruck in dem Reaktor von weniger als oder gleich 7 bar, wobei die flüssige Reaktionszusammensetzung umfaßt (a) einen Rhodiumkatalysator, (b) Alkylhalogenid, (c) mindestens eine endliche Konzentration Wasser und (d) einen Mangan in einer zur Beschleunigung der Carbonylierungsreaktion aktiven Form umfassenden Stabilisator für den Rhodiumkatalysator bei einem Molverhältnis Mangan:Rhodium von (0,2 bis 20):1.

4. Verfahren nach Anspruch 3, wobei der Manganstabilisator in der flüssigen Reaktionszusammensetzung als Mangan(I) vorliegt.

5. Verfahren nach Anspruch 3 oder Anspruch 4, wobei die flüssige Reaktionszusammensetzung Wasser bei einer Konzentration unterhalb 14 Gewichtsprozent, vorzugsweise unterhalb 11 Gewichtsprozent, bevorzugter unterhalb 8 Gewichtsprozent, umfaßt.

6. Verfahren nach einem der Ansprüche 3 bis 5, wobei die flüssige Reaktionszusammensetzung außerdem einen Jodidsalzstabilisator, ausgewählt aus der Gruppe, bestehend aus Metalljodiden, quaternären Ammoniumjodiden und quaternären Phosphoniumjodiden, umfaßt.

7. Verfahren nach Anspruch 6, wobei der Metalljodidsalzstabilisator ausgewählt ist aus der Gruppe, bestehend aus Alkalijodiden und Erdalkalimetalljodiden, vorzugsweise aus der Gruppe, bestehend aus Jodiden von Lithium, Natrium, Kalium und Cäsium; das quaternäre Ammoniumjodid ausgewählt ist aus der Gruppe, bestehend aus quaternisierten Aminen, Pyridinen, Pyrrolidinen und Imidazolen, und vorzugsweise N,N'-Dimethylimidazoliumjodid ist, und das quaternäre Phosphoniumjodid ausgewählt ist aus der Gruppe, bestehend aus Methyltributylphosphoniumjodid, Tetrabutylphosphoniumjodid und Methyltriphenylphosphoniumjodid.

8. Verfahren nach einem der Ansprüche 3 bis 7, wobei das Molverhältnis von Mangan:Rhodium in der flüssigen Reaktionszusammensetzung (0,5 bis 10):1 ist.

9. Verfahren nach einem der Ansprüche 3 bis 8, wobei der Manganstabilisator durch Inkontaktbringen mit Wasserstoff zu einer aktiven Form umgesetzt wird.

10. Verfahren nach einem der vorangehenden Ansprüche, umfassend in einem Carbonylierungsreaktor Inkontaktbringen von Methanol und/oder Essigsäuremethylester mit Kohlenmonoxid in einer flüssigen Reaktionszusammensetzung bei einem Kohlenmonoxid-Partialdruck in dem Reaktor von weniger als oder gleich 7 bar, und wobei die flüssige Reaktionszusammensetzung umfaßt (a) einen Rhodiumkatalysator bei einer Konzentration in der flüssigen Reaktionszusammensetzung von 100 bis 1500 ppm Rhodium auf das Gewicht, (b) 2 bis 16 Gewichtsprozent Methyljodid, (c) weniger als 8 Gewichtsprozent Wasser und (d) einen Mangan in einer zur Beschleunigung der Carbonylierungsreaktion aktiven Form umfassenden Stabilisator für den Rhodiumkatalysator bei einem Molverhältnis Manganstabilisator: Rhodiumkatalysator von (0,5 bis 10):1.

11. Verfahren nach Anspruch 1, umfassend die Schritte (i) in einem Carbonylierungsreaktor Inkontaktbringen des Alkohols und/oder eines reaktiven Derivats davon mit Kohlenmonoxid in einer flüssigen Reaktionszusammensetzung, umfassend (a) einen Rhodiumkatalysator, (b) Alkylhalogenid und (c) mindestens eine endliche Konzentration an Wasser; (ii) Gewinnen des Carbonylierungsprodukts aus der Reaktionszusammensetzung bei einem Kohlenmonoxid-Partialdruck von weniger als oder gleich 7 bar, in Gegenwart eines Mangan umfassenden Stabilisators für den Rhodiumkatalysator bei einem Molverhältnis Mangan:Rhodium von (0,2 bis 20):1, und (iii) Zurückführen des Rhodiumkatalysators und des Manganstabilisators aus Schritt (ii) zu dem Carbonylierungsschritt (i).

12. Verfahren nach Anspruch 11, wobei das Molverhältnis Mangan:Rhodium in Schritt (ii) (0,5 bis 10):1 ist.

13. Verfahren nach Anspruch 11 oder Anspruch 12, wobei der Kohlenmonoxid-Partialdruck in Schritt (ii) weniger als 0,25 bar beträgt.

14. Verfahren nach einem der vorangehenden Ansprüche, wobei der Alkylalkohol Methanol umfaßt und das Carbonylierungsprodukt Essigsäure umfaßt.

## Revendications

1. Procédé pour la carbonylation d'un alcool alkylique et/ou d'un de ses dérivés, procédé qui comprend les étapes consistant (i) à mettre en contact, dans un réacteur de carbonylation, cet alcool et/ou son dérivé réactif avec du monoxyde de carbone dans une composition réactionnelle liquide comprenant (a) un catalyseur au rhodium, (b) un halogénure d'alkyle, et (c) au moins une concentration finie d'eau, et (ii) à séparer le produit de carbonylation de ladite composition réactionnelle liquide, caractérisé en ce qu'il existe dans les étapes de carbonylation et/ou de séparation du produit une pression partielle de monoxyde de carbone inférieure ou égale à 7 bars et un stabilisant pour le catalyseur au rhodium, comprenant du manganèse en un rapport molaire manganèse:rhodium de (0,2 à 20):1.

2. Procédé suivant la revendication 1, dans lequel le rapport molaire manganèse:rhodium est un rapport de (0,5 à 10):1.

3. Procédé suivant la revendication 1, qui comprend la mise en contact, dans un réacteur de carbonylation, d'un alcool et/ou d'un de ses dérivés réactifs avec du monoxyde de carbone dans une composition réactionnelle liquide à une pression partielle de monoxyde de carbone dans ledit réacteur inférieure ou égale à 7 bars, dans lequel la composition réactionnelle liquide comprend (a) un catalyseur au rhodium, (b) un halogénure d'alkyle, (c) au moins une concentration finie d'eau, et (d) un stabilisant pour le catalyseur au rhodium, comprenant du manganèse sous une forme efficace pour activer la réaction de carbonylation en un rapport molaire manganèse:rhodium de (0,2 à 20):1.

4. Procédé suivant la revendication 3, dans lequel le stabilisant à base de manganèse est présent dans la composition réactionnelle liquide sous forme de manganèse (I).

5. Procédé suivant la revendication 3 ou la revendication 4, dans lequel la composition réactionnelle liquide comprend de l'eau à une concentration inférieure à 14 % en poids, avantageusement inférieure à 11 % en poids, de préférence inférieure à 8 % en poids.

6. Procédé suivant l'une quelconque des revendications 3 à 5, dans lequel la composition réactionnelle liquide comprend en outre un stabilisant consistant en iodure choisi dans le groupe consistant en iodures métalliques, iodures d'ammonium quaternaire et iodures de phosphonium quaternaire.

7. Procédé suivant la revendication 6, dans lequel le stabilisant consistant en un iodure métallique est choisi dans le groupe consistant en iodures de métaux alcalins et iodures de métaux alcalino-terreux, de préférence dans le groupe consistant en iodures de lithium, de sodium, de potassium et de césium ; l'iodure d'ammonium quaternaire est choisi dans le groupe consistant en iodures de dérivés quaternaires d'amines, de pyridines, de pyrrolidines et d'imidazoles et est de préférence l'iodure de N,N'-diméthylimidazolium ; et l'iodure de phosphonium quaternaire est choisi dans le groupe consistant en iodure de méthyltributylphosphonium, iodure de tétrabutylphosphonium et iodure de méthyltriphénylphosphonium.

8. Procédé suivant l'une quelconque des revendications 3 à 7, dans lequel le rapport molaire manganèse:rhodium dans la composition réactionnelle liquide est un rapport de (0,5 à 10):1.

9. Procédé suivant l'une quelconque des revendications 3 à 8, dans lequel le stabilisant à base de manganèse est transformé en une forme active par mise en contact avec de l'hydrogène.

10. Procédé suivant l'une quelconque des revendications précédentes, qui comprend la mise en contact, dans un réacteur de carbonylation, de méthanol et/ou d'acétate de méthyle avec du monoxyde de carbone dans une composition réactionnelle liquide à une pression partielle de monoxyde de carbone dans ledit réacteur inférieure ou égale à 7 bars, et dans lequel la composition réactionnelle liquide comprend (a) un catalyseur au rhodium à une concentration dans la composition réactionnelle liquide de 100 à 1500 ppm en poids de rhodium, (b) 2 à 16 % en poids d'iodure de méthyle, (c) moins de 8 % en poids d'eau, et (d) un stabilisant pour le catalyseur au rhodium, comprenant du manganèse sous une forme efficace pour activer la réaction de carbonylation en un rapport molaire stabilisant à base de manganèse:catalyseur au rhodium de (0,5 à 10):1.

11. Procédé suivant la revendication 1, qui comprend les étapes consistant (i) à mettre en contact, dans un réacteur de carbonylation, l'alcool et/ou son dérivé réactif avec du monoxyde de carbone dans une composition réactionnelle liquide comprenant (a) un catalyseur au rhodium, (b) un halogénure d'alkyle, et (c) au moins une concentration finie d'eau, (ii) à séparer le produit de carbonylation de ladite composition réactionnelle à une pression partielle de monoxyde de carbone inférieure ou égale à 7 bars en présence d'un stabilisant pour le catalyseur au rhodium, comprenant du manganèse, en un rapport molaire manganèse:rhodium de (0,2 à 20):1; et (iii) à recycler de l'étape (ii) ledit catalyseur au rhodium et ledit stabilisant à base de manganèse à ladite étape de carbonylation (i).

12. Procédé suivant la revendication 11, dans lequel le rapport molaire manganèse:rhodium dans l'étape (ii) est un rapport de (0,5 à 10):1.

13. Procédé suivant la revendication 11 ou 12, dans lequel la pression partielle de monoxyde de carbone dans l'étape (ii) est inférieure à 0,25 bar.

14. Procédé suivant l'une quelconque des revendications précédentes, dans lequel l'alcool alkylique comprend le méthanol et le produit de carbonylation comprend l'acide acétique.
